# EUROPEAN PATENT SPECIFICATION

(11) **EP 1 492 457 B1**
(45) Date of publication and mention of the grant of the patent: **07.12.2005**
(21) Application number: 04702585.3
(22) Date of filing: 15.01.2004
(51) Int. Cl.: A61B 10/00, B65D 75/26, A61B 19/02

(54) **AUTOMATICALLY OPENING MEDICAL DEVICE PACKAGE AND METHOD OF MANUFACTURE**
VERPACKUNG FÜR MEDIZINISCHE GERÄTE, DIE SICH AUTOMATISCH ÖFFNET, UND HERSTELLUNGSVERFAHREN
EMBALLAGE A OUVERTURE AUTOMATIQUE POUR DISPOSITIF MEDICAL, ET SON PROCEDE DE FABRICATION

(30) Priority: 25.02.2003 US 374440
(43) Date of publication of application: 05.01.2005
(73) Proprietor: LIFESCAN, INC., Milpitas, CA 95035-6312 (US)
(72) Inventor: ALLEN, John, J., Mendota Heights, MN 55118 (US)
(74) Representative: Mercer, Christopher Paul
(86) International application number: PCT/US2004/001237
(87) International publication number: WO 2004/075760

(56) References cited:
- EP-A- 0 500 208
- GB-A- 1 099 920
- US-A- 2 676 702

## Description

### BACKGROUND OF INVENTION

### 1. Field of the Invention

The present invention relates, in general, to medical device packages and, in particular, to automatically opening medical device packages and methods for manufacturing the same.

### 2. Description of the Related Art

A variety of medical devices require packaging to, for example, protect the medical device from damage prior to use and to maintain sterility of the medical device. For medical devices that include a skin-piercing element (e.g., a lancet or micro-needle), an associated package should provide for deployment of the skin-piercing element during use while also protecting a user from inadvertent contact with the skin-piercing element following use. Furthermore, single-use disposable medical devices call for a medical device package that is inexpensive and disposable. Although it is conceivable that rigid injection molded medical device packages could be designed to provide protection of medical devices enclosed therein, it is likely that their cost and potentially cumbersome manual deployment (i.e., opening) procedures would be less than ideal.

Still needed in the field, therefore, is an inexpensive medical device package that does not require cumbersome manual opening procedures yet still provides for protection of a medical device enclosed therein and/or a sterility barrier. Furthermore, for medical devices that include a skin-piercing element (e.g., a lancet or micro-needle), a need exists for a medical device package that protects the skin-piercing element from damage or contamination prior to use and that also protects a user from accidental contact with the skin-piercing element following use.

GB 1,099,920 discloses a medical device package for a skin-piercing element according to the preamble of claim 1.

### SUMMARY OF INVENTION

Automatically opening medical device packages according to embodiments of the present invention are inexpensive, provide protection and a sterility barrier for medical devices enclosed therein and do not require cumbersome manual opening procedures. Furthermore, embodiments of automatically opening packages according to the present invention are suitable for use with medical devices that include a skin-piercing element (e.g., a lancet or micro-needle) since the packages serve to protect the skin-piercing element from damage or contamination prior to use, as well as to protect a user from accidental contact with the skin-piercing element following use.

An automatically opening medical device package according to an exemplary embodiment of the present invention includes upper and lower flexible sheet portions, each having a distal end, a proximal end, a first peripheral edge and a second peripheral edge. The upper and lower flexible sheet portions are adapted to be detachably sealed together along at least a segment of their first and second peripheral edges, thereby providing for the enclosing of a medical device (e.g., a biosensor medical device, a lancet medical device or an integrated lancet and biosensor medical device) within the upper and lower flexible sheet portions.

The package also includes a collar (e.g., a rigid collar) attached to the distal ends of the upper and lower flexible sheet portions. The collar is attached in such a way that a relative movement of the collar and the proximal ends of the upper and lower flexible sheet portions, that decreases a distance therebetween, results in a pulling apart of the upper and lower flexible sheet portions. This pulling apart automatically opens the package and exposes (e.g., deploys) at least a portion (e.g., a skin-piercing element portion) of the medical device.

Also provided by the present invention is a method of manufacturing an automatically opening medical device package containing a medical device (e.g., an integrated biosensor and lancet medical device). The method includes positioning at least one medical device on a flexible sheet of material. The flexible sheet of material on which the medical device(s) is positioned has upper and lower surfaces, and first and second ends, with the medical device(s) being positioned on the upper surface between the first and second ends.

The method also includes attaching an upper collar precursor to the lower surface of the first end of the flexible sheet of material and a lower collar precursor to the lower surface of the second end of the flexible sheet of material. The flexible sheet of material is subsequently folded end-over-end about the medical device(s), thereby forming an upper flexible sheet portion and a lower flexible sheet portion that enclose the medical device(s). Each of the upper and lower flexible sheet portions thus formed has both a distal end and a proximal end. The upper flexible sheet portion is then detachably sealed to the lower flexible sheet portion.

The upper and lower collar precursors are rolled-back across the upper flexible sheet portion and lower flexible sheet portion, respectively, such that the upper and lower collar precursors are operatively aligned. The upper and lower collar precursors are then joined together to form a collar attached to the distal ends of the upper and lower flexible sheet portions. The collar is attached in such a manner that a relative movement of the collar and the proximal ends of the upper and lower flexible sheet portions, that decreases a distance therebetween, results in a pulling apart of the upper and lower flexible sheet portions, thereby automatically exposing at least a portion of the medical device.

### BRIEF DESCRIPTION OF DRAWINGS

A better understanding of the features and advantages of the present invention will be obtained by reference to the following detailed description that sets forth illustrative embodiments, in which the principles of the invention are utilized, and the accompanying drawings, of which:
FIG. 1A is a perspective top view of an exemplary embodiment of an automatically opening medical device package according to the present invention;
FIG. 1B is a perspective bottom view of the package of FIG. 1A that illustrates (with dashed lines) the location of an integrated biosensor and lancet medical device within the package;
FIG. 2A is a perspective view of a the package of FIGs. 1A and 1B following automatic opening of the package;
FIG. 2B illustrates with dashed lines the location of the integrated biosensor and lancet medical device within the opened package of FIG. 2A;
FIG. 3 is a flow chart illustrating a sequence of steps in a process according to an exemplary embodiment of the present invention;
FIGs. 4A and 4B are perspective and side views, respectively, depicting the result of a step in the manufacturing of the package shown in FIG. 1A with FIG. 4B representing a view along line 4B-4B of FIG. 4A;
FIGs. 5A and 5B are perspective and side views, respectively, depicting a result of another step in the manufacturing of the package shown in FIG. 1A with FIG. 5B representing a view along line 5B-5B of FIG. 5A; and
FIGs. 6A and 6B are perspective and side views, respectively, depicting a result of yet another step in the manufacturing of the package shown in FIG. 1A with FIG. 6B representing a view along line 6B-6B of FIG. 6A.

### DETAILED DESCRIPTION OF THE INVENTION

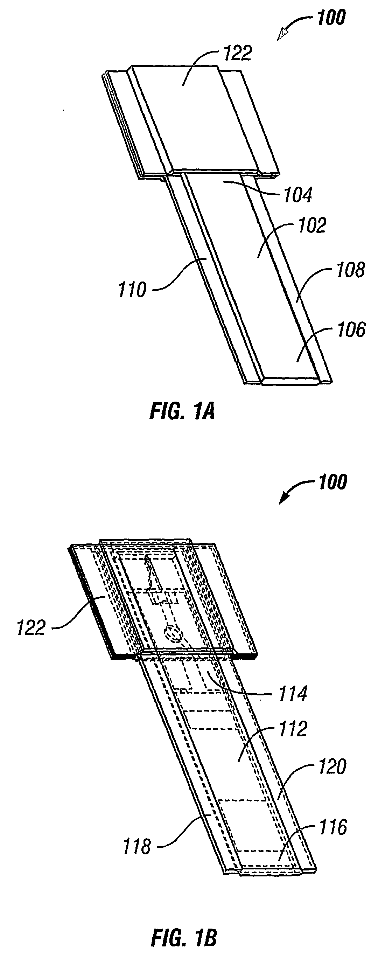

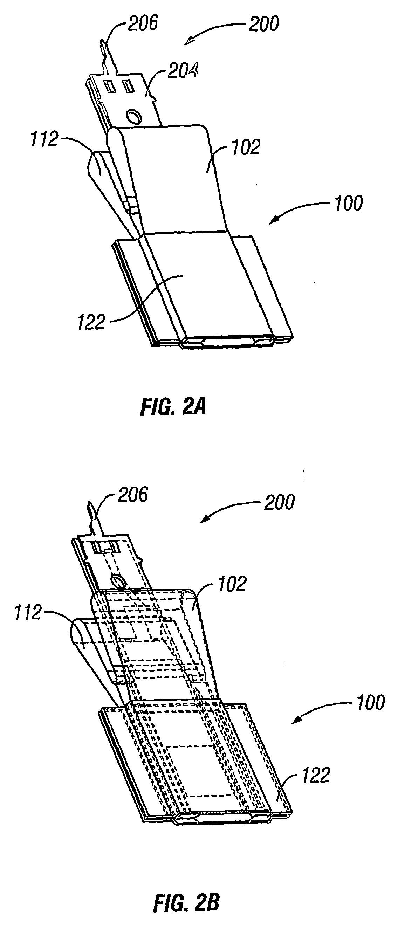

FIG. 1A is a perspective view of an automatically opening medical device package 100 according to an exemplary embodiment of the present invention. FIG. 1B illustrates, with dashed lines, a medical device 200 (i.e., an integrated biosensor 204 and lancet 206 medical device) enclosed within package 100 using a perspective bottom view. FIG. 2A is a perspective view of the package of FIGs. 1A and 1B following automatic opening of the package. FIG. 2B illustrates, with dashed lines, the location of the integrated biosensor and lancet medical device within the opened package of FIG. 2A.

Once apprised of the present disclosure, one skilled in the art will recognize that a variety of medical devices can be beneficially employed with packages according to the present invention. For example, integrated biosensor and lancet medical devices that can be beneficially employed with the current invention are described in U.S. Patent Application No. 10/143,399, which is hereby fully incorporated herein by reference. Moreover, those skilled in the art will recognize that packages according to the present invention are not limited to use with integrated biosensor and lancet medical devices. For example, they can also be used with test strip medical devices of the types described in U. S. patent Nos. 5,426,032, 5,526,120, 5,708,247, 6,241,862 and 6,284,125. Furthermore, one skilled in the art will appreciate that such test strip medical devices are not limited to the measurement of glucose but can also be used to measure, for example, ketones, glycated albumin, coagulation parameters and cholesterol of a sample.

Package 100 includes an upper flexible sheet portion 102 having a distal end 104, a proximal end 106, a first peripheral edge 108 and a second peripheral edge 110, as shown in FIGs. 1A through 2B. Package 100 also includes a lower flexible sheet portion 112 having a distal end 114, a proximal end 116, a first peripheral edge 118 and a second peripheral edge 120. The distal ends, proximal ends, first peripheral edges and second peripheral edges of the upper and lower flexible sheet portions are essentially aligned with one another (as shown in the top and bottom views of FIGs. 1A and 1B).

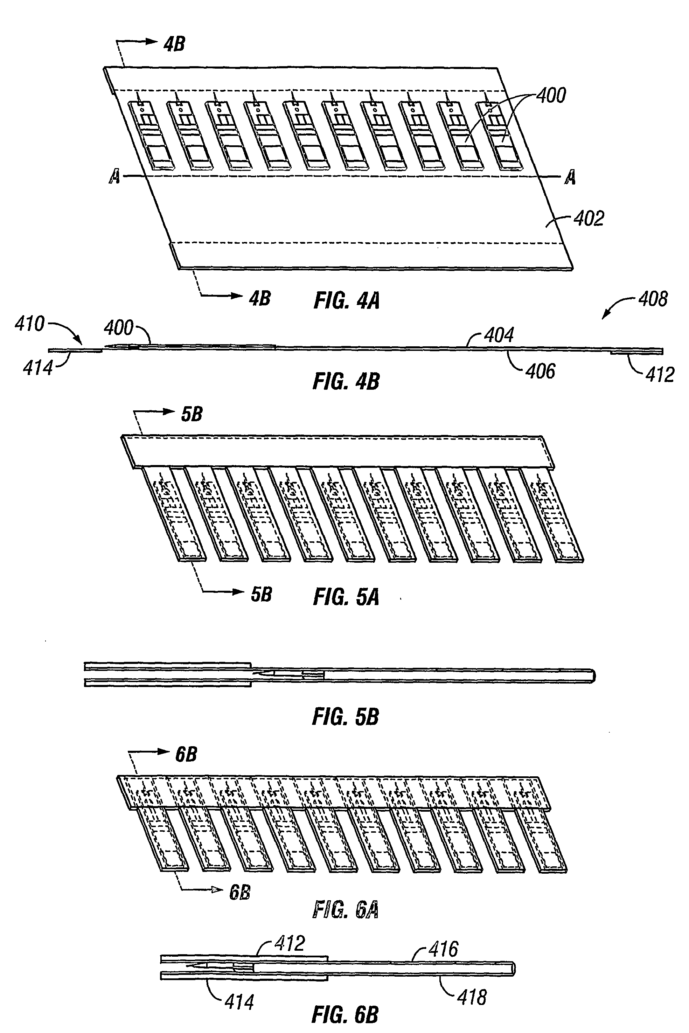

Upper and lower flexible sheet portions 102 and 112 can be formed, for example, from a single sheet of flexible material that has been folded over upon itself (as described further below with respect to FIGs. 4A through 6B). In such a circumstance, proximal ends 106 and 116 are attached to one another along a line where the single sheet of flexible material has been folded.

The upper and lower flexible sheet portions 102 and 112 can be formed from any suitable flexible material known to one skilled in the art. For example, the upper and lower sheet portions can be formed of a flexible paper material (e.g., Kraft Paper, TPP-0036 [HSC 42# Surgical Kraft Paper]), and/or a film-type material (e.g., TPF-0504 [Nylon Film - puncture resistant] and 1073B uncoated Tyvek film). Suitable flexible materials are commercially available from, for example, Tolas Health Care Packaging. Furthermore, if desired, the upper and lower flexible sheet portions can be formed of a flexible laminated material such as, for example, a combination of the flexible paper and film-type materials noted above with a metal foil and/or latex coating.

Upper and lower flexible sheet portions 102 and 112 of package 100 are adapted to be detachably sealed together along at least a segment of their first and second peripheral edges. Any suitable adhesive known to one skilled in the art can be employed for this purpose including, but not limited to, cold-seal adhesives, heat-seal adhesives and releasable adhesives available from, for example, 3M, Basic Adhesives and Avery Dennison. In the embodiment of FIGs. 1A, 1B, 2A and 2B, upper and lower flexible sheet portions 102 and 112 of package 100 are also adapted to be detachably sealed together along at least a segment of their proximal and distal ends such that a complete sterility barrier is provided for medical device 200.

In the embodiment of FIGs. 1A through 2B, the sealing of the upper and lower flexible sheet portions provides for enclosing a medical device (e.g., the integrated biosensor and lancet device illustrated in FIGs. 1BA and 2B) within the upper and lower flexible sheet portions. Once the upper and lower flexible sheet portions have been sealed together around the medical device (for example, by sealing the peripheral edges and, optionally, the distal and proximal ends of the upper and lower flexible sheet portions), a sterility barrier can also be provided.

Package 100 also includes a collar 122 attached to the distal ends (104 and 114) of the upper and lower flexible sheet portions (102 and 122, respectively). Collar 122 is attached to the distal ends of the upper and lower flexible sheet portions in such a manner that a relative movement of the collar and the proximal ends of the upper and lower flexible sheet portions, that decreases a distance therebetween, results in a pulling apart of the lower and upper flexible sheet portions. This pulling apart automatically opens the package and exposes (i.e., deploys) at least a portion (e.g., a skin-piercing element portion) of the medical device that had previously been enclosed entirely within the package. In other words, collar 122 is slid toward proximal ends 106 and 116 (or vice versa), thereby pulling apart the upper and lower flexible sheet portions and deploying the enclosed medical device, as illustrated in FIGs. 2A and 2B.

It is envisioned that the relative movement of the collar and the proximal ends can be achieved in a variety of ways. For example, such movement can be induced by a user upon manually moving the collar and/or proximal ends relative to each other. The relative movement can also occur when a user inserts the package into a suitable mechanical interface (e.g., a analytical meter interface), or it can be imposed by a mechanical device (e.g., an analytical meter).

Collar 122 can be formed of any suitable material including, but not limited to, a suitable rigid material (e.g., a rigid plastic film of Mylar and/or polyester) or semi-rigid material. In this regard, a semi-rigid material is a material that has some flexibility but is significantly less flexibility than the upper and lower flexible sheet portions. Although a collar need not be rigid or semi-rigid, a rigid or semi-rigid collar can serve to provide additional protection for an enclosed medical device. In the embodiment of FIGs. 1A through 2B, collar 122 is a rigid collar and therefore adapted to provide impact protection for the lancet 206 of medical device 200 that is encircled by collar 122. Furthermore, the rigid nature of collar 122 serves to facilitate the pulling apart of the upper and lower flexible sheet portions.

In the embodiment of FIGs. 1A through 2B, attachment of collar 122 to the distal ends of the upper and lower flexible sheet portions is such that a subsequent relative movement of the collar and the proximal ends of the upper and lower flexible sheet portions, that increases the distance therebetween, results in an automatic enclosing of the medical device by the upper and lower flexible sheet portions. Such enclosing thereby retracts at least a portion of the medical device, which had been exposed by the prior pulling apart of the upper and lower flexible sheet portions. In other words, once package 100 has been opened (as illustrated in FIGs. 2A and 2B), package 100 can be returned to the configuration shown in FIGs. 1 A and 1B by moving collar 122 away from the proximal ends 106 and 116 (or vise versa). Once collar 122 has been returned to the position of FIGs. 1A and 1B, package 100 covers lancet 206 for disposal and protects a user from inadvertent contact with lancet 206.

Automatically opening medical device packages according to the present invention are inexpensive, have a relatively slim profile, and provide a sterility barrier and protection from inadvertent contact with a medical device enclosed therein. Furthermore, humidity protection can also be provided by forming the upper and lower flexible sheet portions from a moisture resistant material (e.g., a metal foil material) and labeling can optionally be applied to the surfaces of the upper and lower flexible sheet portions.

Those skilled in the art will also recognize that automatically opening medical device packages according to the present invention can be secondarily packaged for single use in, for example, a vial or in a cartridge configured for dispensing the automatically opening medical device packages. The secondary package may be constructed of material containing desiccant or may contain separately packaged desiccant for keeping contents moisture free.

Embodiments of the present invention also encompass an automatically opening medical device package and medical device kit that includes automatically opening medical device packages as described above and a medical device (e.g., an integrated biosensor and lancet medical device for measuring blood glucose).

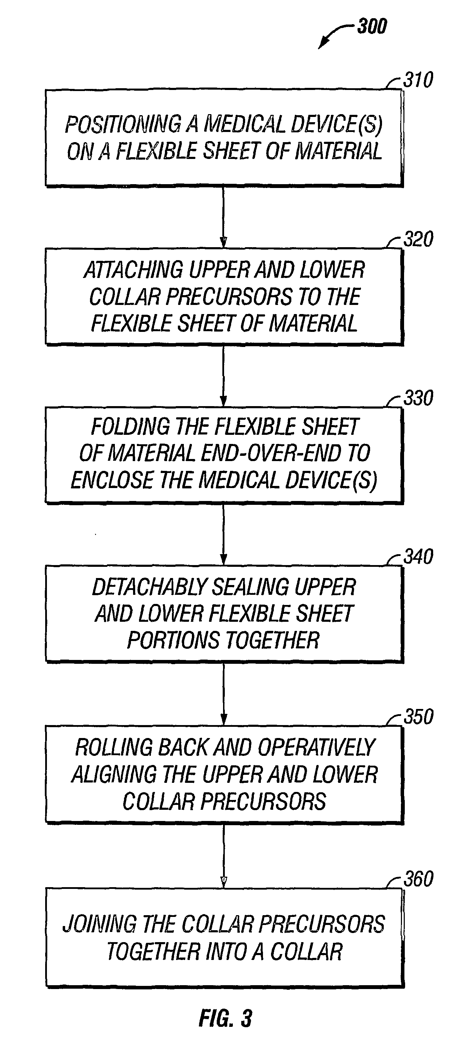

FIG. 3 is a flow chart illustrating a sequence of steps in a process 300 for manufacturing an automatically opening medical device package containing a medical device according to an exemplary embodiment of the present invention. One skilled in the art will recognize that process 300 can be implemented using, for example, web-based manufacturing techniques and other well known conventional manufacturing techniques.

Referring to FIG. 3, FIG. 4A and FIG. 4B, process 300 includes positioning at least one medical device (e.g., an integrated biosensor and lancet medical device 400) on a flexible sheet of material 402, as set forth in step 310 of FIG. 3. Flexible sheet of material 402 can be formed of any suitable material known to one skilled in the art. If desired to provide a sterility barrier for medical device 400, the flexible sheet of material 402 can be formed from a flexible material that is impervious to air and/or air-borne bacteria, such as a thermoplastic flexible material and a metallic foil material.

In the embodiment of FIGs. 4A and 4B, flexible sheet of material 402 has an upper surface 404, a lower surface 406, a first end 408 and a second end 410. In the embodiment of FIGs. 4A through 6B, ten integrated biosensor and lancet devices 400 are positioned on flexible sheet of material 402. In positioning step 310, the medical device(s) is positioned on the upper surface 404 between the first and second ends 408 and 410, respectively.

Next, an upper collar precursor 412 is attached to the lower surface of the first end 408 of the flexible sheet of material and a lower collar precursor 414 is attached to the lower surface of the second end 410 of the flexible sheet of material, as set forth in step 320. The locations of upper and lower collar precursors 412 and 414 are indicated by dashed lines in FIGs. 4A and 5A. Upper and lower collar precursors 412 and 414 can be formed of any suitable material including, for example, rigid materials such as cardboard. The result of steps 310 and 320 of process 300 is depicted in FIG. 4A and FIG. 4B.

Subsequent to the attachment of the upper and lower collar precursors, the flexible sheet of material is folded end-over-end about the medical device(s), thereby forming an upper flexible sheet portion 416 and a lower flexible sheet portion 418 enclosing the medical device(s) 400, as set forth in step 330 of FIG. 3. Folding step 320 can occur, for example, along line A-A of Fig. 4A. Portions of the flexible sheet material between medical devices 400 are then removed. The resultant structure is illustrated in FIGs. 5A and 5B.

Next, the upper flexible sheet portion and lower flexible sheet portion are detachably sealed (using, for example, a heat or pressure sensitive adhesive) to provide protective enclosures for medical devices 400, as set forth in step 340 of FIG. 3. Alternatively, the detachable sealing can occur prior to the removal of portions of the flexible sheet material between medical devices 400.

The upper and lower collar precursors are then rolled-back across the upper flexible sheet portion and lower flexible sheet portion respectively, such that the upper and lower collar precursors 412 and 414 are operatively aligned, as set forth in step 350. The resultant structure is illustrated in FIGs. 6A and 6B.

Next, as set forth in step 360 of FIG. 3, the upper and lower collar precursors are joined together (for example, along outside edges of the upper and lower collar precursors) to form a collar attached to the distal ends of the upper and lower flexible sheet portions in such a way that a relative movement of the collar and the proximal ends of the upper and lower flexible sheet portions, that decreases a distance therebetween, results in a pulling apart of the upper and lower flexible sheet portions. This pulling apart, thereby, automatically exposes and deploys at least a portion of the medical device.

In the circumstance that a plurality of medical devices were positioned on the flexible sheet of material in step 310, each medical device and associated package is singulated by cutting through the collar formed by joining the upper and lower collar precursors. Alternatively, the medical devices and associate packages can be singulated prior to step 360 of FIG. 3.

One skilled in the art will recognize that processes according to the present invention are inexpensive and readily implemented using conventional manufacturing techniques (e.g., web-based manufacturing techniques).

It should be understood that various alternatives to the embodiments of the invention described herein may be employed in practicing the invention. It is intended that the following claims define the scope of the invention and that structures and methods within the scope of these claims be covered thereby.

## Claims

1. An automatically opening medical device package (100), the package comprising:
an upper flexible sheet portion (102) having a distal end (104), a proximal end (106), a first peripheral edge and a second peripheral edge;
a lower flexible sheet portion (112) having a distal end (114), a proximal end (116), a first peripheral edge and a second peripheral edge,
wherein the upper and lower flexible sheet portions are adapted to be detachably sealed together along at least a segment of their first (108) and second (110) peripheral edges, thereby providing for the enclosing of a medical device (200) within the upper and lower flexible sheet portions; **characterised in that** a collar (122) is attached to the distal end of the upper flexible sheet portion and the distal end of the lower flexible sheet portion,
wherein the collar is attached to the distal ends of the upper and lower flexible sheet portions in a manner such that a relative sliding movement of the collar and the proximal ends of the upper and lower flexible sheet portions, that decreases a distance therebetween, results in a pulling apart of the lower and upper flexible sheet portions, thereby automatically opening the package and exposing at least a portion of the medical device.

2. The package of claim 1, wherein the upper (102) and lower (112) flexible sheet portions are adapted to enclose an integrated biosensor and lancet medical device (200).

3. The package of claim 1, wherein the upper (102) and lower (112) flexible sheet portions are adapted to enclose a biosensor medical device.

4. The package of claim 1, wherein the upper (102) and lower (112) flexible sheet portions are adapted to enclose a lancet medical device.

5. The package of claim 1, wherein the collar (122) encircles a portion of a medical device enclosed within the upper (102) and lower (112) flexible sheet portions.

6. The package of claim 1, wherein the collar (122) is made of a rigid material.

7. The package of claim 6, wherein the collar (122) is made of cardboard.

8. The package of claim 6, wherein the collar (122) is made of a plastic film.

9. The package of claim 1, wherein the collar (122) is made of a semi-rigid material.

10. The package of claim 1, wherein the proximal ends (106, 116) of the upper (102) and lower (112) flexible sheet portions are joined together.

11. The package of claim 1, wherein the upper (102) and lower (112) flexible sheet portions are portions of a single unitary flexible sheet.

12. The package of claim 1, wherein the upper (102) and lower (112) flexible sheet portions are formed of paper.

13. The package of claim 1, wherein the upper (102) and lower (112) flexible sheet portions are formed of a film.

14. The package of claim 1, wherein the upper (102) and lower (112) flexible sheet portions are formed of plastic.

15. The package of claim 1, wherein the upper (102) and lower (112) flexible sheets are detachably sealed using an adhesive coating.

16. The package of claim 1, wherein the collar (122) is attached to the distal ends (104, 114) of the upper (102) and lower (112) flexible sheet portions such that, following the relative movement of the collar (122) and the proximal ends (106, 116) of the upper and lower flexible sheet portions that decreases a distance therebetween, a subsequent relative movement of the collar and proximal ends of the upper and lower flexible sheets, that increases the distance therebetween, results in an automatic enclosing of the medical device by the upper and lower flexible sheet portions.

17. The package of claim 16, wherein the automatic enclosing results in a re-sealing of the upper and lower flexible sheet portions.

18. An automatically opening medical device package and medical device kit, the kit comprising:
a package according to any one of claims 1 to 17; and
a medical device (200) contained within the package.

19. The kit of claim 18, wherein the medical device (200) is an integrated biosensor and lancet medical device.

20. The kit of claim 19, wherein the integrated biosensor and lancet medical device is adapted for the measurement of blood glucose.

21. A method for manufacturing an automatically opening medical device package according to claim 1 containing a medical device, the method comprising:
positioning at least one medical device on a flexible sheet of material, the flexible sheet of material having an upper surface, a lower surface, a first end and a second end, the at least one medical device being positioned on the upper surface between the first and second ends;
attaching an upper collar precursor to the lower surface of the first end of the flexible sheet of material and a lower collar precursor to the lower surface of the second end of the flexible sheet of material;
folding the flexible sheet of material end-over-end about the at least one medical device, thereby forming an upper flexible sheet portion and a lower flexible sheet portion enclosing the at least one medical device, each of the upper and lower flexible sheet portions having a distal end and a proximal end;
detachably sealing the upper flexible sheet portion to the lower flexible sheet portion;
rolling-back the upper and lower collar precursors across the upper flexible sheet portion and lower flexible sheet portion, respectively, such that the upper and lower collar precursors are operatively aligned; and
joining the upper and lower collar precursors together to form a collar attached to the distal ends of the upper and lower flexible sheet portions in a manner such that a relative movement of the collar and the proximal ends of the upper and lower flexible sheet portions, that decreases a distance therebetween, results in a pulling apart of the upper and lower flexible sheet portions, thereby automatically exposing at least a portion of the medical device.

22. The method of claim 22, wherein the detachably sealing step forms a sterility barrier.

23. The method of claim 22, wherein the positioning step includes positioning a plurality of medical devices.

24. The method of claim 22 further comprising the step of removing portions of the flexible sheet of material after the sealing step and before the rolling-back step.

25. The method of claim 22, wherein the positioning step includes positioning at least one integrated biosensor and lancet medical device.

## Patentansprüche

1. Automatisch öffnende Verpackung (100) für medizinische Geräte, die Folgendes umfaßt:
einen oberen flexiblen Flächenabschnitt (102), der ein distales Ende (104), ein proximales Ende (106), einen ersten peripheren Rand und einen zweiten peripheren Rand aufweist;
einen unteren flexiblen Flächenabschnitt (112), der ein distales Ende (114), ein proximales Ende (116), einen ersten peripheren Rand und einen zweiten peripheren Rand aufweist;
wobei der obere und der untere flexible Flächenabschnitt dazu geeignet sind, daß sie entlang zumindest eines Segments ihrer ersten (108) und zweiten (110) peripheren Ränder lösbar miteinander versiegelt werden können, wodurch ein medizinisches Gerät (200) innerhalb des oberen und des unteren flexiblen Flächenabschnitts umschlossen wird; **dadurch gekennzeichnet, daß**
an dem distalen Ende des oberen flexiblen Flächenabschnitts und an dem distalen Ende des unteren flexiblen Flächenabschnitts ein Bund (122) angebracht ist, wobei der Bund an den distalen Enden des unteren und oberen flexiblen Flächenabschnitts in einer Weise angebracht ist, daß eine relative, abgleitende Bewegung des Bundes und der proximalen Enden des oberen und des unteren flexiblen Flächenabschnitts, die einen Abstand dazwischen verringert, dazu führt, daß sich ein Auseinanderziehen des oberen und des unteren flexiblen Flächenabschnitts ergibt, wodurch automatisch die Verpackung geöffnet wird und zumindest ein Abschnitt des medizinischen Gerätes freigelegt wird.

2. Verpackung nach Anspruch 1, **dadurch gekennzeichnet, daß**
der obere (102) und der untere (112) flexible Flächenabschnitt dazu geeignet sind, ein integriertes medizinisches Biosensor- und Lanzetten-Gerät (200) zu umschließen.

3. Verpackung nach Anspruch 1, **dadurch gekennzeichnet, daß**
der obere (102) und der untere (112) flexible Flächenabschnitt dazu geeignet sind, ein medizinisches Biosensor-Gerät zu umschließen.

4. Verpackung nach Anspruch 1, **dadurch gekennzeichnet, daß**
der obere (102) und der untere (112) flexible Flächenabschnitt dazu geeignet sind, ein medizinisches Lanzetten-Gerät zu umschließen.

5. Verpackung nach Anspruch 1, **dadurch gekennzeichnet, daß**
der Bund (122) einen Abschnitt eines medizinischen Gerätes, das innerhalb des oberen (108) und des unteren (112) flexiblen Flächenabschnitts umschlossen ist, umgibt.

6. Verpackung nach Anspruch 1, **dadurch gekennzeichnet, daß**
der Bund (122) aus einem rigiden Material besteht.

7. Verpackung nach Anspruch 6, **dadurch gekennzeichnet, daß**
der Bund (122) aus Pappe besteht.

8. Verpackung nach Anspruch 6, **dadurch gekennzeichnet, daß**
der Bund (122) aus einem Kunststofffilm besteht.

9. Verpackung nach Anspruch 1, **dadurch gekennzeichnet, daß**
der Bund (122) aus einem semi-rigiden Material besteht.

10. Verpackung nach Anspruch 1, **dadurch gekennzeichnet, daß**
die proximalen Enden (106, 116) des oberen (102) und des unteren (112) flexiblen Flächenabschnitts aneinandergefügt sind.

11. Verpackung nach Anspruch 1, **dadurch gekennzeichnet, daß**
der obere (102) und der untere (112) flexible Flächenabschnitt Abschnitte aus einer einzelnen einheitlichen flexiblen Fläche sind.

12. Verpackung nach Anspruch 1, **dadurch gekennzeichnet, daß**
der obere (102) und der untere (112) flexible Flächenabschnitt aus Papier gestaltet sind.

13. Verpackung nach Anspruch 1, **dadurch gekennzeichnet, daß**
der obere (102) und der untere (112) flexible Flächenabschnitt aus einem Film gestaltet sind.

14. Verpackung nach Anspruch 1, **dadurch gekennzeichnet, daß**
der obere (102) und der untere (112) flexible Flächenabschnitt aus Kunststoff gestaltet sind.

15. Verpackung nach Anspruch 1, **dadurch gekennzeichnet, daß**
die obere (102) und die untere (112) flexible Fläche mittels einer adhäsiven Beschichtung lösbar versiegelt sind.

16. Verpackung nach Anspruch 1, **dadurch gekennzeichnet, daß**
der Bund (122) an den distalen Enden (104, 114) des oberen (102) und des unteren (112) flexiblen Flächenabschnitts in einer solchen Weise angebracht ist, daß nach der relativen Bewegung des Bundes (122) und der proximalen Enden (106, 116) des oberen und des unteren flexiblen Flächenabschnitts, die einen Abstand dazwischen verringert, eine nachfolgende relative Bewegung des Bundes und der proximalen Enden des oberen und des unteren flexiblen Flächenabschnitts, die den Abstand dazwischen verändert, dazu führt, daß das medizinische Gerät automatisch durch den oberen und den unteren flexiblen Flächenabschnitt umschlossen wird.

17. Verpackung nach Anspruch 16, **dadurch gekennzeichnet, daß**
das automatische Umschließen zu einer erneuten Versiegelung des oberen und des unteren flexiblen Flächenabschnitts führt.

18. Automatisch öffnende Verpackung für medizinische Geräte und eine medizinische Geräteeinrichtung, wobei die Einrichtung Folgendes umfaßt:
eine Verpackung nach einem der Ansprüche 1 bis 17; und
ein medizinisches Gerät (200), das in der Verpackung enthalten ist.

19. Einrichtung nach Anspruch 18, **dadurch gekennzeichnet, daß**
es sich bei dem medizinischen Gerät (200) um ein integriertes medizinisches Biosensor- und Lanzetten-Gerät handelt.

20. Einrichtung nach Anspruch 19, **dadurch gekennzeichnet, daß**
das integrierte medizinische Biosensor- und Lanzetten-Gerät für die Messung des Blutzuckers geeignet ist.

21. Verfahren zur Herstellung einer automatisch öffnenden Verpackung für medizinische Geräte nach Anspruch 1, die ein medizinisches Gerät enthält, wobei das Verfahren Folgendes umfaßt:
Anordnen zumindest eines medizinischen Gerätes auf einer flexiblen Materialfläche, wobei diese flexible Materialfläche eine obere Oberfläche, eine untere Oberfläche, ein erstes Ende und ein zweites Ende aufweist, wobei das zumindest eine medizinische Gerät auf der oberen Oberfläche zwischen dem ersten und dem zweiten Ende angeordnet ist;
Anbringen eines oberen Bund-Vorläufers an der unteren Oberfläche des ersten Endes der flexiblen Materialfläche und eines unteren Bund-Vorläufers an der unteren Oberfläche des zweiten Endes der flexiblen Materialfläche;
Falten der flexiblen Materialfläche Ende über Ende um das zumindest eine medizinische Gerät, wodurch ein oberer flexibler Flächenabschnitt und ein unterer flexibler Flächenabschnitt entstehen, die das zumindest eine medizinische Gerät umschließen, wobei sowohl der obere als auch der untere flexible Flächenabschnitt ein distales Ende und ein proximales Ende aufweisen;
Lösbares Versiegeln des oberen flexiblen Flächenabschnitts an dem unteren flexiblen Flächenabschnitt in lösbarer Weise;
Zurückrollen des oberen und des unteren Bund-Vorläufers über den oberen flexiblen Flächenabschnitt und den unteren flexiblen Flächenabschnitt jeweils in einer solchen Weise, daß der obere und der untere Bund-Vorläufer wirksam ausgerichtet sind; und
Zusammenfügen des oberen und unteren Bund-Vorläufers, um einen Bund zu bilden, der an den distalen Enden des oberen und unteren flexiblen Flächenabschnitts in einer solchen Weise angebracht ist, daß eine relative Bewegung des Bundes und der proximalen Enden des oberen und des unteren flexiblen Flächenabschnitts in einer solchen Weise, daß ein Abstand dazwischen verringert wird, dazu führt, daß sich ein Auseinanderziehen des oberen und des unteren flexiblen Flächenabschnitts ergibt, wodurch automatisch zumindest ein Abschnitt des medizinischen Gerätes freigelegt wird.

22. Verfahren nach Anspruch 22, **dadurch gekennzeichnet, daß**
der Schritt des lösbaren Versiegelns eine Sterilitätsbarriere bildet.

23. Verfahren nach Anspruch 22, **dadurch gekennzeichnet, daß**
der Schritt des Anordnens das Anordnen mehrerer medizinischer Geräte enthält.

24. Verfahren nach Anspruch 22, **dadurch gekennzeichnet, daß**
das Verfahren ferner den Schritt des Entfernens von Abschnitten der flexiblen Materialfläche nach dem Schritt der Versiegelung und vor dem Schritt des Zurückrollens umfaßt.

25. Verfahren nach Anspruch 22, **dadurch gekennzeichnet, daß**
der Schritt des Anordnens das Anordnen zumindest eines integrierten medizinischen Biosensor- und Lanzetten-Gerätes beinhaltet.

## Revendications

1. Emballage à ouverture automatique pour dispositif médical (200), l'emballage comprenant :
une partie de feuille flexible supérieure (102) présentant une extrémité distale (104), une extrémité proximale (106), un premier bord périphérique et un deuxième bord périphérique ;
une partie de feuille flexible inférieure (112) présentant une extrémité distale (114), une extrémité proximale (116), un premier bord périphérique et un deuxième bord périphérique ;
dans lequel les parties de feuille flexible supérieures et inférieures sont adaptées pour être scellées ensemble de manière détachable le long d'au moins un segment de leurs premier (109) et deuxième (110) bords périphériques, fournissant de ce fait l'insertion d'un dispositif médical (200) dans les parties de feuille flexible supérieures et inférieures ; **caractérisé en ce que**
un collier (122) est attaché à l'extrémité distale de la partie de feuille flexible supérieure et à l'extrémité distale de la partie de feuille flexible inférieure ;
le collier est attaché aux extrémités distales des parties de feuille flexible supérieures et inférieures de sorte qu'un mouvement de glissement relatif du collier et des extrémités proximales des parties de feuille flexible supérieures et inférieures, qui réduit la distance entre celles-ci, entraîne un démontage des parties de feuille flexible supérieures et inférieures, ouvrant de ce fait automatiquement l'emballage et exposant au moins une partie du dispositif médical.

2. Emballage selon la revendication 1, dans lequel les parties de feuille flexible supérieures (102) et inférieures (112) sont adaptées pour enfermer un dispositif médical à lancette et biocapteur intégrés.

3. Emballage selon la revendication 1, dans lequel les parties de feuille flexible supérieures (102) et inférieures (112) sont adaptées pour enfermer un dispositif médical à biocapteur (200).

4. Emballage selon la revendication 1, dans lequel les parties de feuille flexible supérieures (102) et inférieures (112) sont adaptées pour enfermer un dispositif médical à lancette.

5. Emballage selon la revendication 1, dans lequel le collier (122) encercle une partie d'un dispositif médical enfermé dans les parties de feuille flexible supérieures (102) et inférieures (112).

6. Emballage selon la revendication 1, dans lequel le collier (122) est composé d'un matériau rigide.

7. Emballage selon la revendication 6, dans lequel le collier (122) est composé de carton.

8. Emballage selon la revendication 6, dans lequel le collier (122) est composé d'un film plastique.

9. Emballage selon la revendication 1, dans lequel le collier (122) est composé d'un matériau semi-rigide.

10. Emballage selon la revendication 1, dans lequel les extrémités proximales (106, 116) des parties de feuille flexible supérieures (102) et inférieures (112) sont jointes.

11. Emballage selon la revendication 1, dans lequel les parties de feuille flexible supérieures (102) et inférieures (112) sont des parties de feuille flexible unitaire simple.

12. Emballage selon la revendication 1, dans lequel les parties de feuille flexible supérieures (102) et inférieures (112) sont composées de papier.

13. Emballage selon la revendication 1, dans lequel les parties de feuille flexible supérieures (102) et inférieures (112) sont composées d'un film.

14. Emballage selon la revendication 1, dans lequel les parties de feuille flexible supérieures (102) et inférieures (112) sont composées de plastique.

15. Emballage selon la revendication 1, dans lequel les parties de feuille flexible supérieures (102) et inférieures (112) sont scellées de manière détachable à l'aide d'un revêtement adhésif.

16. Emballage selon la revendication 1, dans lequel le collier (122) est attaché aux extrémités distales (104, 114) des parties de feuille flexible supérieures (102) et inférieures (112) de sorte que, en suivant le mouvement relatif du collier (122) et des extrémités proximales (106, 116) des parties de feuille flexible supérieures et inférieures qui réduit la distance entre celles-ci, un mouvement relatif ultérieur du collier et des extrémités proximales des feuilles flexible supérieures et inférieures, qui augmente la distance entre celles-ci, entraîne l'insertion automatique d'un dispositif médical par les parties de feuille flexible supérieures et inférieures.

17. Emballage selon la revendication 16, dans lequel l'insertion automatique entraîne de nouveau le scellage des parties de feuille flexible supérieures et inférieures.

18. Emballage à ouverture automatique pour dispositif médical et un jeu de dispositif médical, le jeu comprenant :
un emballage, selon l'une quelconque des revendications 1 à 17 ;
un dispositif médical (200) contenu dans l'emballage.

19. Jeu selon la revendication 18, dans lequel le dispositif médical (200) est un dispositif médical à lancette et biocapteur intégrés.

20. Jeu selon la revendication 19, dans lequel le dispositif médical à lancette et biocapteur intégrés est adapté pour mesurer le glucose dans le sang.

21. Procédé de fabrication d'un emballage à ouverture automatique pour dispositif médical selon la revendication 1, contenant un dispositif médical, le procédé comprenant les étapes consistant à :
positionner au moins un dispositif médical sur une feuille flexible de matériau, la feuille flexible de matériau présentant une surface supérieure, une surface inférieure, une première extrémité et une deuxième extrémité, le au moins un dispositif médical étant positionné sur la surface supérieure entre les première et deuxième extrémités ;
fixer un précurseur de collier supérieur à la surface inférieure de la première extrémité de la feuille flexible de matériau et un précurseur de collier inférieur à la surface inférieure de la deuxième extrémité de la feuille flexible de matériau ;
plier la feuille flexible de matériau d'une extrémité à l'autre autour d'au moins un dispositif médical, formant de ce fait une partie de feuille flexible supérieure et une partie de feuille flexible inférieure enfermant le au moins un dispositif médical, chacune des parties de feuille flexible supérieures et inférieures présentant une extrémité distale et une extrémité proximale ;
sceller de manière détachable la partie de feuille flexible supérieure sur la partie de feuille flexible inférieure ;
reculer les précurseurs de collier supérieurs et inférieurs à travers la partie de feuille flexible supérieure et la partie de feuille flexible inférieure, respectivement, de sorte que les précurseurs de collier supérieurs et inférieurs sont alignés de manière opérationnelle ; et
joindre les précurseurs supérieurs et inférieurs pour former un collier fixé aux extrémités distales des parties de feuille flexible supérieures et inférieures de sorte qu'un mouvement relatif du collier et des extrémités proximales des parties de feuille flexible supérieures et inférieures, qui diminue une distance entre celles-ci, entraîne un démontage des parties de feuilles flexible supérieures et inférieures, exposant de ce fait automatiquement au moins une partie du dispositif médical.

22. Procédé selon la revendication 22, dans lequel l'étape consistant à sceller de manière détachable forme une barrière stérile.

23. Procédé selon la revendication 22, dans lequel l'étape de positionnement comprend le positionnement d'une pluralité de dispositifs médicaux.

24. Procédé selon la revendication 22 comprenant en outre l'étape consistant à retirer les parties de feuille flexible de matériau après l'étape consistant à sceller et avant l'étape de recul.

25. Procédé selon la revendication 22, dans lequel l'étape de positionnement comprend le positionnement d'au moins un dispositif médical à lancette et biocapteur intégrés.
